# EUROPEAN PATENT SPECIFICATION

(11) **EP 1 407 266 B1**
(45) Date of publication and mention of the grant of the patent: **17.02.2010**
(21) Application number: 02780858.3
(22) Date of filing: 24.06.2002
(51) Int. Cl.: G01N 33/53

(54) **METHOD FOR ESTIMATION OF THE AMOUNT OF SPECIFIC NEUTROPHIL CELL TYPES**
VERFAHREN ZUR ABSCHÄTZUNG DER ANZAHL AN SPEZIFISCHEN NEUTROPHILEN ZELLARTEN
PROCEDE D'EVALUATION D'UNE QUANTITE DE TYPES DE CELLULES SPECIFIQUES NEUTROPHILES

(30) Priority: 25.06.2001 SE 0102220
(43) Date of publication of application: 14.04.2004
(73) Proprietor: Phadia AB, 751 37 Uppsala (SE)
(72) Inventor: VENGE, Per, S-753 12 Uppsala (SE)
(74) Representative: Lindgren, Anders
(86) International application number: PCT/SE2002/001214
(87) International publication number: WO 2003/001205

(56) References cited:
- DATABASE MEDLINE [Online] METSO TUULA ET AL.: 'Cell specific markers for eosinophils and neutrophils in sputum and bronchoalveolar lavage fluid of patients with respiratory conditions and healthy subjects', XP002957464 Retrieved from NCBI Database accession no. 11978925 & THORAX vol. 57, no. 5, May 2002, pages 449 - 451
- AMIN K. ET AL.: 'Eosinophils and neutrophils in biopsies from the middle ear of atopic children with otitis media with effusion' INFLAMM. RES. vol. 48, 1999, pages 626 - 631, XP002957465
- DATABASE MEDLINE [Online] KARAWAJCZYK M. ET AL.: 'The differential release of eosinophil granule proteins. Studies on patients with acute vacterial and viral infections', XP002957466 Retrieved from NCBI Database accession no. 7584682 & CLINICAL AND EXPERIMENTAL ALLERGY: JOURNAL OF THE BRITISH SOCIETY FOR ALLERGY AND CLINICAL IMMUNOLOGY vol. 25, no. 8, August 1995, pages 713 - 719
- DATABASE MEDLINE [Online] SKUBITZ K.M. ET AL.: 'Preparation and characterization of monoclonal antibodies to human neutrophil cathepsin G, lactoferrin, eosinophil peroxidase and eosinophil major basic protein', XP000971423 Retrieved from NCBI Database accession no. 2746138 & JOURNAL OF LEUKOCYTE BIOLOGY vol. 46, no. 2, August 1989, pages 109 - 118
- SCHNEIDER THORSTEN ET AL.: 'Quantitation of eosinophil and neutrophil infiltration into rat lung by specific assays for eosinophil peroxidase and myeloperoxidase' JOURNAL OF IMMUNOLOGICAL METHODS vol. 198, 1996, pages 1 - 14, XP004021305

## Description

### Field of the invention

The present invention relates to the estimation of the amount of subtypes of specific cells, for example the number of certain subtypes of leukocytes, by measurements of unique proteins in extracts of blood and other biological material. The knowledge of the number or amount of specific subtypes of white cells is important in the clinical diagnosis and surveillance of subjects with inflammatory disease including infectious disease, cancer, allergy/asthma etc.

### Background of the invention

The estimation of the number of various leukocytes in blood and other body fluids is one of the most widely used tools in medicine. The traditional way of obtaining this information is the counting and differentiating of the cells under the light microscope. This technique is complemented by the automated counting in cell counters based on the principle of counting the number of particles in the fluid and the measurement of various physical parameters such as size, forward and side scatter, but also my histochemical staining of cells. An extension of these techniques is the flow cytometer principle in which antibodies are used to identify individual cells based on their cell surface antigens or by means of their content of intracellular antigens after permeabilisation of the cells.

In WO 00/58726 there is described a method for quantitating leukocyte count in whole blood. However, this method does not quantitate different specific subtypes of leukocytes in respect of number or ratio.

### Summary of the invention

There exists a need of easy-to-use, inexpensive and reliable tests to estimate the number of various white bloods cells such as neutrophils and eosinophils, in blood and other body fluids, applicable in the point-of-care situation, thus supporting the medical doctor in his/hers immediate decision-making.

The present inventor has found that the extraction of whole blood with for example detergents such as CTAB (N-cetyl-*N*,*N*,*N*-trimethylammonium bromide), and the subsequent measurement by specific immunoassays of the neutrophil proteins, MPO (mycloperoxidase), HNL (human neutrophil lipocalin) or lactoferrin, or the specific measurements of eosinophil proteins such as EPX (eosinophil protein x) or EPO (eosinophil peroxidase), will accurately identify the number of or eosinophils present in the blood. The estimation of the numbers of neutrophils is useful in the diagnosis and monitoring of subjects with inflammatory diseases such as infections, rheumatoid diseases, but also in conjunction with medical treatment, in particular cytostatic treatment, where the reduced production of neutrophils i.e. neutropenia, may occur as a serious adverse effect of treatment. The estimation of eosinophil numbers is useful in patients with allergic disease, chronic inflammatory diseases, parasitic disease, certain cancers such as Hodgkin's disease, but also as a general indicator of disease, since elevated numbers of eosinophils may occur in a number of diseases for unknown reasons.

The invention refers to the estimation of populations at various maturation stages of myeloid cells, since some intracellular proteins are produced primarily by immature cells and other proteins primarily by more mature cells.

Thus, the invention relates to a method to in vitro estimate the amount, wherein the amount refers to either the number or ratio of specific cell subtypes in a patient sample, comprising
a) extracting an aliquot of said sample; and
b) measuring the concentration of two cell specific molecules in said extracted sample by an immunological assay and determining a ratio between the concentrations of said molecules,
wherein the cell specific molecules are neutrophil proteins.

The sample is preferably blood or other body fluid. A very small amount of sample is extracted, such as 1-10 µl of sample but larger volumes might be considered.

The extraction is preferably with cationic detergent. The extraction time is very short, for example 1 minute. Preferably, the detergent is CTAB.

Preferably, the measuring in step b) is by an immunoassay, such as ELISA, EIA, FEIA, RIA.

The cell specific molecule(s) are neutrophil proteins such as MPO (myeloperaxidase), HNL (human neutrophil lipocalin) or lactoferrin for measuring neutrophils.

### Detailed description of the invention

The present invention will be described more closely below in association with the accompanying drawings in which
Fig. 1 shows a correlation between the number of blood neutrophils and the concentration of MPO protein in detergent extracted whole blood.
Fig. 2 shows ratio between MPO and lactoferrin in detergent extracted whole blood.
Fig. 3 shows a correlation between the number of eosinophils and the concentration of EPO protein in detergent extracted whole blood.

### EXPERIMENTAL SECTION

### ISOLATION AND PURIFICATION OF HUMAN NEUTROPHIL AND EOSINOPHIL GRANULE PROTEINS.

Granules were prepared from the buffy coat of granulocytes obtained from healthy blood donors using a modification of the procedure described by Peterson et al (Eur. J. Haematol. 40 (1988) 415-423). In brief, the red blood cells were allowed to sediment using Dextran T-500 before collection of the leukocyte rich plasma. Leucocytes were washed twice in 0,34 M Sucrose and the suspended in 5 volumes of 0,34 M Sucrose. The leukocytes were cavitated using N₂ at a pressure of 750 psi for 30 min at +4 °C (Klempner et al., J. Cell. Biol. 86 (1980) 21-28; and Borregaard et al., .J. Cell. Biol. 97 (1983) 52-61). The cavitate was suspended in 0,34 M Sucrose, 0,17 M NaCl and centrifuged for 20 min at 450xg at +4 °C
The supernatant was centrifuged for 20 min at 10,000 x g at +4 °C to sediment the granules. Myeloperoxidase (MPO) was purified from granule extracts according Olsson et al (Scand. J. Haematol. 9 (1972) 483-491) and Cooray et al (Vet. Immunol. Immunopathol. 38 (1993) 261-272). The final preparation was completely homogenous according to the absorbance ratio A430 mn/A280 nm which was 0,80 (Agner Acta. Chem. Scand. 12 (1958) 89-94.
Human neutrophil lipocalin (HNL) was purified as described (Xu et al., Scand J Clin Lab Invest 54 (1994) 365-376. HNL was purified to homogeneity according SDS-PAGE electrophoresis and silver staining and the antigen did not react with antibodies against the other neutrophil proteins, MPO; Lactoferrin, Cathepsin G, Elastase and Lysozyme. Lactoferrin was purified as described (Reiter Int. J. Tissue React. 5 (1983) 87-96.
Eosinophil Peroxidase (EPO) was purified as described (Carlson et al J. Immunol. 134 (1985) 1875-1879) and the final preparation was homogenous according to the absorbance ratio A415 nm/A280 nm that was 1,15.
Eosinophil protein (EPX) was purified to homogeneity as described (Peterson et al., Immunol. 50 (1983) 19-26. The final preparation appeared as one band on SDS-PAGE electrophoresis and did not react with antibodies against eosinophil cationic protein (ECP), elastase, cathepsin G, MPO and EPO.

### PRODUCTION OF ANTIBODIES

### Polyclonal antibodies

Antibodies against MPO, HNL, EPO and EPX was raised in rabbits by multiple site intracutaneous injections into the rabbits of total 50-100 µg of the purified proteins suspended in Freund's complete and incomplete adjuvant The specificity of the antibodies was evaluated by double immuno diffussion (Ouchterlony Acta Pathol. Microbiol. Scand 26 (1949) 507-) in agarose and tested against extracts of neutrophils and eosinophil granules and the following purified proteins: cathepsin G, elastase, MPO, lysozyme, lactoferrin, ECP, EPX; EPO.

### Monoclonal antibodies

Female Balb/c mice were immunized subcutaneously with purified protein. Priming was done by injecting 50 µg of pure protein mixed with Freund's complete adjuvant. Three boosters were done with approximately 50 µg of pure protein in PBS (phosphate buffered saline).Spleen cells were fused as described (Galfré et al., Nature 266 (1977) 550-552) with Sp2/0 myeloma cells. Supernatants from the cell cultures were screened for antibodies using ELISA technique with antigen-coated wells. Antibodies in supernatants were also screened for specificity to respective granule protein and mapped for epitopes in BIAcore® (BIAcore, Uppsala, Sweden). Hybridomas were selected according to the ELISA and BIAcore experiments and cloned, expanded and purified. All selected antibodies were of IgG1 subtype.

### Immunoassays

HNL was assessed using a radioimmunoassay as described (Xu et al., J. Immunol. Methods 171 (1994) 245-252. Inter- and intra assay variations were less than 10 % and detection limit was less than 4 µg/l.
EPX and Myeloperoxidase was measured using commercially available radioimmunoassays (Pharmacia Diagnostics AB, Uppsala, Sweden). Inter- and intra assay variations were less than 10 % and detection limit was less than 3 and 8 µg/l, respectively
EPO was measured using a protype immunofluorometric assay utilising the Pharmacia CAP system® as described (Nielsen et al., Allergy 53 (1998) 778-785. Inter- and intra assay variations were less than 8 % and detection limit was less than 0,5 µg/l.
Lactoferrin was estimated as described (Olofsson et al., Scand J Haematol 18 (1977) 73-80). Inter- and intra assay variations were less than 8 % and detection limit was less than 2 µg/l.

### Blood samples

EDTA-containing blood samples drawn from patients were randomly collected at the University Hospital, Uppsala, Sweden. Blood cell counts were performed on each sample by means of a Coulter STKS (Beckman Coulter, Inc.) cell counter.
Granule proteins were extracted from granulocytes by means of adding CTAB (N-cetyl-*N*,*N*,*N* trimethyl-ammonium bromide) at a final concentration of 0.05-0.5% to a small aliquot of blood, 1-10 µl. The mixture was then incubated for at least 1 minute and then stored frozen at -20°C before analysis.

### Statistical evaluation

Regression analysis was performed using the statistical package, Statistica (Statsoft, Tulsa, USA).

### Example 1: Estimation of the number of blood neutrophils:

The present invention shows that extraction of a small aliquot of blood, 1-10 µl, with CTAB, final concentration 0.05-0.5%, for at least 1 minute and subsequent measurement of the neutrophil protein MPO by means of a specific immunoassay accurately estimates the numbers of neutrophils in the blood.

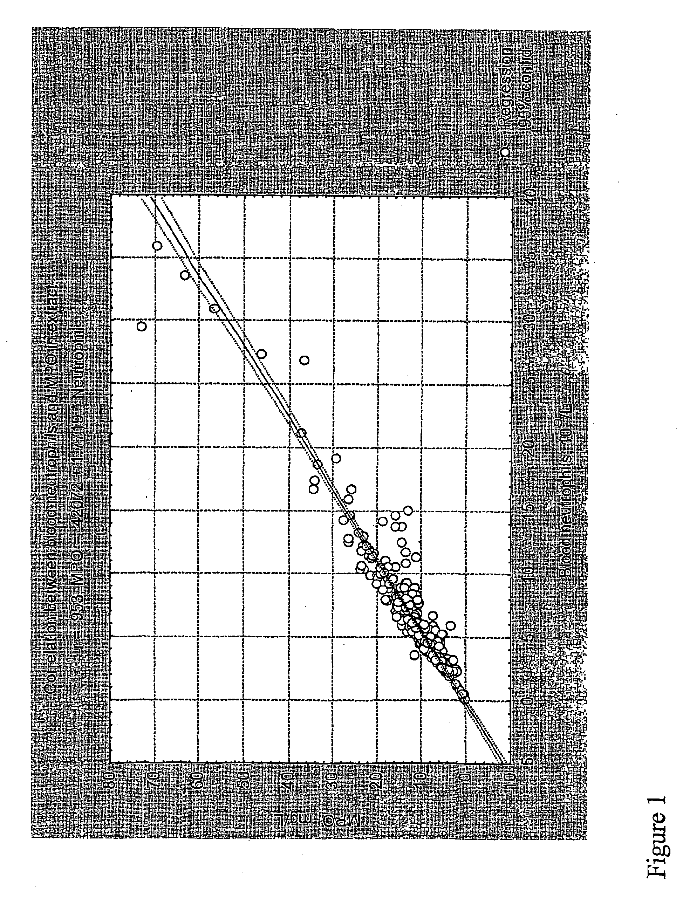

As shown in figure 1, the concentration of MPO in the extract was significantly and linearly correlated (r=0.96) to the number of neutrophils in the extracted blood as estimated by means of a Coulter STKS (Beckman Coulter, Inc.) cell counter. From the equation of the regression line it is apparent that the deviation from origo was minimal, indicating the cell specificity of the measurement. The results were obtained from a mixed population of hospitalised patients (n=275) having both elevated and reduced levels of neutrophils in the their blood. Thus, some patients had highly elevated levels due to acute bacterial infections and others had seriously reduced levels due to leukaemia or cytostatic drug treatment. In spite of the inclusion of these extremes in the calculation, the relationship between number of neutrophils and the concentration of MPO was linear over the entire range measured. When HNL was measured the corresponding correlation was r=0.93 and also with a linear relationship to the number of neutrophils over the entire range. Lactoferrin measurement also showed a linear relationship over the entire range and a correlation coefficient of r=0.82.

### Example 2: Estimation of the degree of maturation of the neutrophil population:

It is well known that MPO is stored in the primary granules of neutrophils, whereas lactoferrin and HNL are stored in secondary granules. This is because the production of MPO primarily takes place during the early maturation steps i.e. by myeloblasts and promyelocytes, whereas lactoferrin and HNL primarily are produced during later maturation steps i.e. by myelocytes. It is also known that production of MPO is less affected by an increased requirement of neutrophils in the circulation, such as in acute infections, than the production of lactoferrin and HNL. The ratio between the content of either of the secondary granule proteins and MPO would therefore provide us with an estimate of the relative size of the various maturation stages of neutrophils in the blood and an indication of the bone marrow turnover of neutrophils.

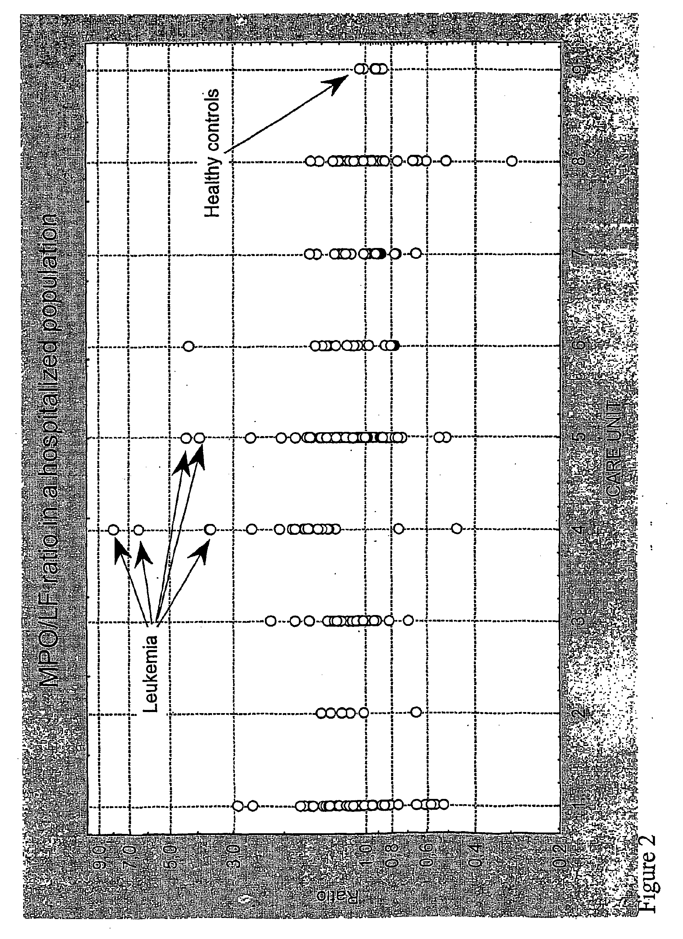

It is shown in fig 2 that a ratio between MPO concentration and lactoferrin concentration in extracted whole blood varies about 20-fold between patients, with myeloid leukaemia patients having the highest ratios.

### Example 3: Estimation of the number of blood eosinophils:

In this example it is shown that extraction of a small aliquot of blood, 1-10 µl, with CTAB, 0.05-0.5%, for at least 1 minute and the subsequent measurement of the eosinophil protein EPO by means of a specific immunoassay accurately estimates the numbers of eosinophils in the blood.

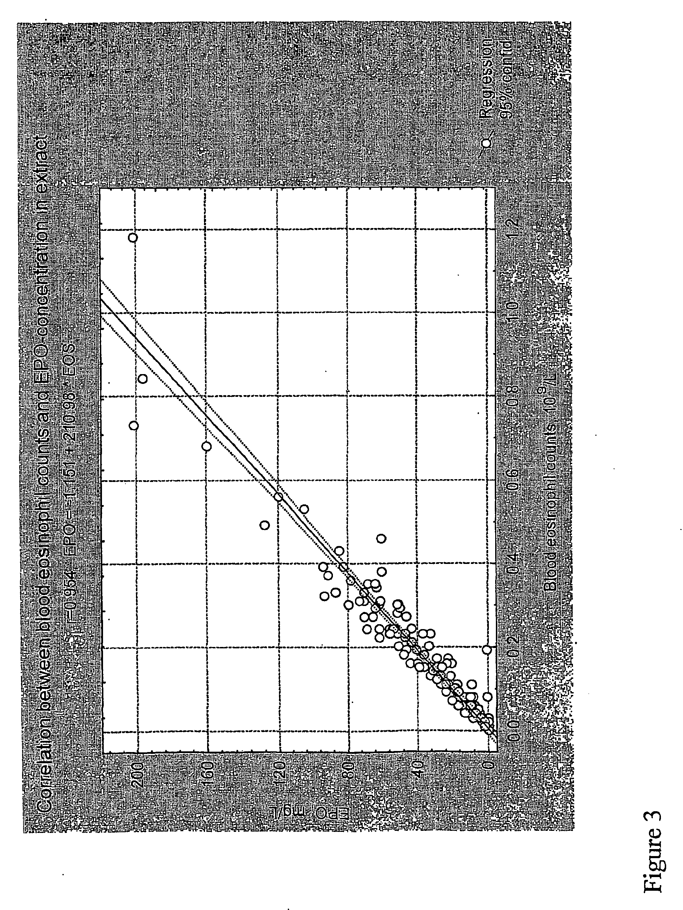

As shown in figure 3, the concentration of EPO in the extract was significantly and linearly correlated (r=0.95) to the number of eosinophils in the extracted blood as estimated by means of a Coulter STKS (Beckman Coulter, Inc.) cell counter. From the equation of the regression line it is apparent that the deviation from origo was minimal, indicating the cell specificity of the measurement. The results were obtained from a mixed population of hospitalised patients (n=275) having both elevated and reduced levels of eosinophils in the their blood. Thus, some patients had elevated numbers because of allergy and asthma, chronic inflammatory diseases, cancer etc. and some had reduced numbers because of, among other things, acute infections.
In spite of the inclusion of these extremes in the calculation, the relationship between number of eosinophils and the concentration of EPO was linear over the entire range measured. When EPX was measured the corresponding correlation was r=0.93 and also with a linear relationship to the number of eosinophils over the entire range.

The above examples 1-3 describe neutrophils, different maturation forms of neutrophils, and eosinophils. However the invention is not to be construed as limited to these cell types.
For example the basophil protein BB1 may measure basophils.
Cell surface markers such as CD20 may measure B-lymphocytes and CD3 T-lymphocytes. The cell surface markers CD4 and CD8 may be used to measure different lymphocyte populations.
Monocytes may be measured by CD14 or lysozyme.
Thrombocytes may be measured by β-tromboglobulin

Determination of ratios is especially interesting for myeloid cells as described in Example 2, but also for various subpopulations of lymphocytes.

### References

Agner K. Crystalline myeloperoxidase. Acta Chem Scand 1958; 12:89-94.
Borregaard N, Heiple JM, Simons ER et al. Subcellular localization of the b-cytochrome component of the human neutrophil microbicidal oxidase: translocation during activation. J. Cell. Biol. 1983;97:52-61
Carlson MGCh, Peterson CGB, Venge P. Human eosinophil peroxidase: Purification and characterization. J Immunol 1985; 134:1875-9.
Cooray R, Petersson CGB, Holmberg O. Isolation and purification of bovine myeloperoxidase from neutrophil granules. Vet Immunol Immunopathol 1993; 38:261-72.
Galfré G, Howe SC, Milstein C et al. Antibodies to major histocompatibility antigens produced by hybrid cell lines. Nature 1977;266:550-552
Klempner MS, Mikkelsen RB, Corfman DH et al. Neutrophil plasma membranes. I. High-yield purification of human neutrophil plasma membrane vesicles by nitrogen cavitation and differential centrifugation.et al., J Cell Biol 1980 Jul;86(1):21-28.
Nielsen LP, Bjerke T, Christensen MB et al. Eosinophil markers in seasonal allergic rhinitis. Intranasal fluticasone propionate inhibits local and systemic increases during the pollen season. Allergy 1998; 53:778-85.
Olofsson T, Olsson I, Venge P et al. Serum myeloperoxidase and lactoferrin in neutropenia. Scand J Haematol 1977; 18(1):73-80.
Olsson I, Olofsson T, Odeberg H. Myeloperoxidase-mediated iodination in granulocytes. Scand J Haematol 1972; 9(5):483-91.
Oucterlony Ö. Antigen antibody reactions in gels. Acta Pathol Microbiol Scand 1949;26:507-.
Peterson CGB, Jörnvall H, Venge P. Purification and characterization of eosinophil cationic protein from normal human eosinophils. Eur J Haematol 1988; 40:415-23.
Peterson CGB, Venge P. Purification and characterization of a new cationic protein-eosinophil protein-X (EPX) - from granules of human eosinophils. Immunol 1983; 50:19-26.
Reiter B. The biological significance of lactoferrin. Int J Tissue React 1983; 5:87-96.
Xu SY, Carlson M, Engström et al. Purification and characterization of a human neutrophil lipocalin (HNL) from the secondary granules of human neutrophils. Scand J Clin Lab Invest 1994; 54:365-76.
Xu SY, Peterson CGB, Carlson M et al. The development of an assay for human neutrophil lipocalin (HNL) - to be used as a specific marker of neutrophil activity in vivo and vitro. J Immunol Methods 1994; I71:245-52.

## Claims

1. A method to in vitro estimate the amount of specific cells in a patient sample, wherein cell specific molecules are used to calculate the specific cell count number, comprising
a) extracting an aliquot of said sample; and
b) measuring the concentration of two cell specific molecules in said extracted sample by an immunological assay and determining a ratio between the concentrations of said molecules,
wherein the cell specific molecules are neutrophil proteins.

2. A method according to claim 1, wherein the extraction is with cationic detergent.

3. A method according to claim 2, wherein the detergent is CTAB (N-cetyl-*N*,*N*,*N-*trimethylammonium bromide).

4. A method according to any of the above claims, wherein the patient sample is whole blood.

5. A method according to any of the above claims, wherein the cell specific molecules are MPO (myeloperoxidase), HNL (human neutrophil lipocalin) or lactoferrin.

## Patentansprüche

1. Verfahren *zur in vitro*-Bestimmung der Menge an spezifischen Zellen in einer Patientenprobe, wobei Zell-spezifische Moleküle verwendet werden, um die spezifische Zellzahl auszurechnen, umfassend
(a) das Extrahieren eines Aliquots der Probe; und
(b) das Messen der Konzentration von zwei Zell-spezifischen Molekülen in der extrahierten Probe mittels eines immunologischen Tests und das Ermitteln eines Verhältnisses zwischen den Konzentrationen der Moleküle,
wobei die Zell-spezifischen Moleküle neutrophile Proteine sind.

2. Verfahren gemäß Anspruch 1, wobei die Extraktion mit kationischem Detergenz geschieht.

3. Verfahren gemäß Anspruch 2, wobei das Detergenz CTAB (N-Cetyl-*N*,*N*,*N-*trimethylammoniumbromid) ist.

4. Verfahren gemäß einem der vorstehenden Ansprüche, wobei die Patientenprobe Vollblut ist.

5. Verfahren gemäß einem der vorstehenden Ansprüche, wobei die Zell-spezifischen Moleküle MPO (Myeloperoxidase), HNL (menschliches neutrophiles Lipocalin) oder Lactoferrin sind.

## Revendications

1. Procédé pour estimer *in vitro* la quantité de cellules spécifiques dans un échantillon de patient, dans lequel des molécules spécifiques à des cellules sont utilisées pour évaluer le nombre de cellules spécifiques comptées, comprenant
a) l'extraction d'une fraction aliquote dudit échantillon ; et
b) la mesure de la concentration par un essai immunologique de deux molécules spécifiques à des cellules dans ledit échantillon extrait et la détermination d'un rapport entre les concentrations desdites molécules,
dans lequel les molécules spécifiques à des cellules sont des protéines neutrophiles.

2. Procédé selon la revendication 1, dans lequel l'extraction est effectuée avec un détergent cationique.

3. Procédé selon la revendication 2, dans lequel le détergent est le CTAB (bromure de N-cétyl-*N*,*N*,*N-*triméthylammonium).

4. Procédé selon l'une quelconque des revendications précédentes, dans lequel l'échantillon de patient est du sang entier.

5. Procédé selon l'une quelconque des revendications précédentes, dans lequel les molécules spécifiques à des cellules sont la MPO (myéloperoxydase), la HNL (lipocaline neutrophile humaine) ou la lactoferrine.
